(19) Europäisches Patentamt
European Patent Office
Office européen des brevets

(11) **EP 4 684 876 A1**

(12) **EUROPEAN PATENT APPLICATION**
published in accordance with Art. 153(4) EPC

(43) Date of publication:
**28.01.2026  Bulletin 2026/05**

(21) Application number: **24774677.9**

(22) Date of filing: **06.03.2024**

(51) International Patent Classification (IPC):
*B01J 35/60* (2024.01)          *B01J 23/888* (2006.01)
*B01J 37/00* (2006.01)          *C07C 51/235* (2006.01)

(52) Cooperative Patent Classification (CPC):
**B01J 23/888; B01J 35/60; B01J 37/00;
C07C 51/235**

(86) International application number:
**PCT/JP2024/008478**

(87) International publication number:
**WO 2024/195536 (26.09.2024 Gazette 2024/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.03.2023  JP 2023046098**

(71) Applicant: **Nippon Kayaku Kabushiki Kaisha
Tokyo 100-0005 (JP)**

(72) Inventors:
• **YASUDA Shogo**
  **Sanyoonoda-shi, Yamaguchi 757-8686 (JP)**
• **OKUMURA Shigeki**
  **Sanyoonoda-shi, Yamaguchi 757-8686 (JP)**

(74) Representative: **Gille Hrabal
Partnerschaftsgesellschaft mbB
Patentanwälte
Brucknerstraße 20
40593 Düsseldorf (DE)**

(54) **CATALYST FOR PRODUCING UNSATURATED CARBOXYLIC ACID**

(57)    The present disclosure relates to a catalyst for producing an unsaturated carboxylic acid having a catalytically active component having a composition represented by the following formula (1) in which the value of the surface roughness S obtained by measuring the surface roughness with a non-contact shape measuring laser microscope is 11.7 $\mu$m or more and 25.0 $\mu$m or less.

$$(Mo)_{12}(V)_a(W)_b(Cu)_c(Sb)_d(X)_e(Y)_f(Z)_g(O)_h \qquad (1)$$

EP 4 684 876 A1

**Description**

TECHNICAL FIELD

The present invention relates to a catalyst for producing an unsaturated carboxylic acid in a high yield by subjecting an unsaturated aldehyde to catalytic gas-phase oxidation in the presence of molecular oxygen or a molecular oxygen-containing gas, a production method therefor, and a method for producing an unsaturated carboxylic acid using the catalyst.

BACKGROUND ART

Acrylic acid is becoming increasingly important as a raw material for a water absorbent resin, an adhesive, and the like. Thus, in recent years, improved performance of a catalyst for producing acrylic acid by subjecting acrolein as a raw material to a catalytic gas-phase oxidation reaction has been demanded. Accordingly, various companies have made various improvements to catalysts that can be used to produce acrylic acid in a high yield and in a stable manner over a long period of time, and for example, the following proposals have been made.
PTLs 1 to 3 propose improvement in the catalyst composition or the like focusing on X-ray diffraction peaks of the catalytically active component. The catalysts disclosed in these documents have been proposed as catalysts that achieve a high activity and a high yield. Further, in PTLs 4 and 5, an improvement guideline for the purpose of improving the mechanical strength of the catalyst is shown, and the catalyst performance is improved through prevention of powdering during filling. In PTL 6, by adjusting the standard deviation of the particle diameter of the catalyst within a specific range, the long-term stability of the catalytic reaction is improved. PTL 7 proposes to produce a catalyst having both high catalyst performance and mechanical strength by controlling the relative centrifugal acceleration during formation using a tumbling granulator. PTL 8 proposes a method for producing a catalyst having a high yield by controlling the density of the molded catalyst body and the roughness of the catalyst surface.
However, particularly in production of an unsaturated carboxylic acid, improvement of the catalytic activity is an important issue. The advantages obtained by improving the catalytic activity are not limited to simple improvement in the yield. For example, when the temperature of the reaction bath at the initial stage of the reaction decreases due to the improvement of the catalytic activity in a plant using a catalyst, the energy cost for heating decreases, and degradation of the performance is small from a long-term viewpoint because the thermal deterioration of the catalyst is reduced. As a result, stable operation and a high yield can be achieved over a long period of time.
For the above reasons, a highly active catalyst and a method for producing the same are required.

CITATION LIST

PATENT LITERATURE

PTL 1: JPH08-299797A
PTL 2: JP2003-251184A
PTL 3: JP2015-120133A
PTL 4: JP2001-79408A
PTL 5: WO2012/073584
PTL 6: JP2009-214105A
PTL 7: JP2015-96497A
PTL 8: WO2020/196150

SUMMARY OF INVENTION

TECHNICAL PROBLEM

In view of the above circumstances, an object of the invention of the present application is to improve the catalytic activity of a catalyst for producing an unsaturated carboxylic acid by a catalytic gas-phase oxidation reaction from an unsaturated aldehyde as a raw material.

# EP 4 684 876 A1

SOLUTION TO PROBLEM

As a result of intensive studies on the current situation and the object described above, the inventors of the present application have found that a specific catalyst shape parameter contributes to improvement in activity and made the invention of the present application.

That is, the present invention relates to the following 1) to 7).

1) A catalyst for producing an unsaturated carboxylic acid with a catalytically active component having a composition represented by the following formula (1) in which the value of the surface roughness S obtained by measuring the surface roughness with a non-contact shape measuring laser microscope is 11.7 $\mu$m or more and 25.0 $\mu$m or less:

$$(Mo)_{12}(V)_a(W)_b(Cu)_c(Sb)_d(X)_e(Y)_f(Z)_g(O)_h \qquad (1)$$

(in the formula (1), Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of niobium, cerium, tin, chromium, manganese, iron, cobalt, samarium, germanium, titanium, and arsenic, a, b, c, d, e, f, g, and h represent the atomic ratios of the respective elements, where a represents $0 < a \leq 10.0$, b represents $0 \leq b \leq 10.0$, c represents $0 < c \leq 6.0$, d represents $0 \leq d \leq 10.0$, e represents $0 \leq e \leq 0.50$, f represents $0 \leq f \leq 1.0$, and g represents $0 \leq g < 6.0$, with respect to molybdenum atom being 12, and h is the number of oxygen atoms necessary for satisfying the valence of each component.)

2) The catalyst for producing an unsaturated carboxylic acid according to 1) above in which in the formula (1), $1.0 \leq a \leq 5.0$, $0.50 \leq b \leq 3.0$, $0.50 \leq c \leq 3.0$, and $0 < d \leq 2.0$ are satisfied.

3) The catalyst for producing an unsaturated carboxylic acid according to 1) or 2) above in which the catalytically active component is carried on an inert carrier.

4) The catalyst for producing an unsaturated carboxylic acid according to 3) above in which the inert carrier is silica, alumina, or a combination thereof.

5) A method for producing the catalyst for producing an unsaturated carboxylic acid according to any one of 1) to 4) above having a step of forming by granulation in which the humidity ratio MR in a granulator is 0.0235 kg-$H_2$O/kg-DA or less.

6) A method for producing an unsaturated carboxylic acid using the catalyst for producing an unsaturated carboxylic acid according to any one of 1) to 4) above.

7) A method for producing an unsaturated carboxylic acid using a reaction tube filled with two or more types of the catalyst for producing an unsaturated carboxylic acid according to any one of 1) to 4) above in multiple layers.

ADVANTAGEOUS EFFECTS OF INVENTION

According to the present invention, a catalyst having an excellent catalytic activity for producing an unsaturated carboxylic acid by subjecting an unsaturated aldehyde as a raw material to a catalytic gas-phase oxidation reaction can be provided.

DESCRIPTION OF EMBODIMENTS

[Arithmetic Average Roughness of Surface of Catalyst Formed Body]

The catalyst of the present invention has surface roughness S of 11.7 $\mu$m or more and 25.0 $\mu$m or less when the surface roughness is measured with a non-contact shape measuring laser microscope.

The surface roughness S of the catalyst can be measured by, for example, a shape analysis laser microscope VK-X1000 (manufactured by KEYENCE) or the like, is a value determined from the number N of the measurement points at which the height of the surface is measured by applying a laser to the catalyst surface and moving in the horizontal direction, the height Zn at each measurement point, and the average value X of the heights at all the measurement points and can be calculated by the following equation (A).

[Equation (A)]

[Math 1]

3

$$S = \frac{1}{N}\sum_{n=1}^{N}|Z_n - X| \cdot \cdot \cdot \quad (A)$$

The upper limit of the surface roughness S is further preferably 24.0 $\mu$m, 23.0 $\mu$m, 22.5 $\mu$m, 22.0 $\mu$m, 21.0 $\mu$m, 20.5 $\mu$m, 20.0 $\mu$m, 19.5 $\mu$m, 19.0 $\mu$m, 18.0 $\mu$m, or 15.0 $\mu$m in this order, and particularly preferably 13.0 $\mu$m. The lower limit is further preferably 11.8 $\mu$m, 12.0 $\mu$m, or 12.3 $\mu$m in this order, and particularly preferably 12.5 $\mu$m. Therefore, the range of S is preferably 11.8 $\mu$m or more and 24.0 $\mu$m or less, more preferably 11.8 $\mu$m or more and 23.0 $\mu$m or less, more preferably 11.8 $\mu$m or more and 22.5 $\mu$m or less, more preferably 11.8 $\mu$m or more and 22.0 $\mu$m or less, more preferably 11.8 $\mu$m or more and 21.5 $\mu$m or less, more preferably 11.8 $\mu$m or more and 21.0 $\mu$m or less, more preferably 11.8 $\mu$m or more and 20.5 $\mu$m or less, more preferably 11.8 $\mu$m or more and 20.0 $\mu$m or less, more preferably 11.8 $\mu$m or more and 19.5 $\mu$m or less, more preferably 11.8 $\mu$m or more and 19.0 $\mu$m or less, more preferably 12.0 $\mu$m or more and 18.0 $\mu$m or less, more preferably 12.3 $\mu$m or more and 15.0 $\mu$m or less, and most preferably 12.5 $\mu$m or more and 13.0 $\mu$m or less.

The inventors of the present invention have found that the catalyst exhibits higher activity as the surface of the catalyst becomes less uneven in a certain range (as the surface roughness S becomes smaller in a certain range). It is believed that, when a catalyst having small unevenness on the catalyst surface is used for a catalytic reaction in which a gas flows in a reaction tube filled with the catalyst, the gas flows without disturbance in the gas flow and without retention in the catalyst layer, and thus the raw material and the product are smoothly exchanged around the catalyst, resulting in improvement of the conversion rate of the raw material. In addition, as another factor, it is believed that, when a catalyst having small unevenness on the catalyst surface is freely dropped from the upper portion of the reaction tube for filling, the catalyst particles are less likely to be caught in each other or in the wall surface of the reaction tube during falling, and the catalyst is filled more densely, resulting in improvement of the conversion rate of the raw material.

On the other hand, when the unevenness of the catalyst surface is too small, the retention of the gas in the reaction tube becomes too short, and the contact time with the catalyst is reduced, resulting in a decrease in the conversion rate of the raw material.

[Catalyst Composition]

The catalyst according to the present invention has a composition represented by the following formula (1).
[Formula (1)]

$$(Mo)_{12}(V)_a(W)_b(Cu)_c(Sb)_d(X)_e(Y)_f(Z)_g(O)_h \qquad (1)$$

(In the formula, Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; and Z represents at least one element selected from the group consisting of bismuth, tellurium, silver, selenium, silicon, aluminum, boron, niobium, cerium, tin, chromium, manganese, iron, cobalt, nickel, samarium, germanium, zirconium, titanium, chromium, tantalum, lead, indium, sulfur, palladium, gallium, lanthanum, and arsenic. a, b, c, d, e, f, g, and h represent the atomic ratios of the respective elements, where a represents $0 < a \le 10.0$, b represents $0 \le b \le 10.0$, c represents $0 < c \le 6.0$, d represents $0 \le d \le 10.0$, e represents $0 \le e \le 0.50$, f represents $0 \le f \le 1.0$, and g represents $0 \le g < 6.0$, with respect to molybdenum atom being 12. Moreover, h is the number of oxygen atoms necessary for satisfying the valence of each component.)

In the above formula (1), preferred ranges of a to g are as follows.

The lower limit of a is 0.20, 0.50, 0.80, 1.0, 1.5, 2.0, 2.2, or 2.5 in order of desirability and is most desirably 2.8, and the upper limit of a is 9.0, 8.0, 7.0, 6.0, 5.0, 4.5, 4.0, or 3.5 in order of desirability and is most desirably 3.2. That is, the range of a is preferably $0.20 \le a \le 9.0$, more preferably $0.50 \le a \le 8.0$, more preferably $0.80 \le a \le 7.0$, more preferably $1.0 \le a \le 6.0$, more preferably $1.5 \le a \le 5.0$, more preferably $2.0 \le a \le 4.5$, more preferably $2.2 \le a \le 4.0$, more preferably $2.5 \le a \le 3.5$, and most preferably $2.8 \le a \le 3.2$.

The lower limit of b is 0.10, 0.20, 0.30, 0.40, 0.50, 0.60, 0.70, 0.80, or 0.90 in order of desirability and is most desirably 1.0, and the upper limit of b is 9.0, 8.0, 7.0, 6.0, 5.0, 4.0, 3.0, 2.5, 2.0, or 1.5 in order of desirability and is most desirably 1.4. That is, the range of b is preferably $0.10 \le b \le 9.0$, more preferably $0.10 \le b \le 8.0$, more preferably $0.20 \le b \le 7.0$, more preferably $0.30 \le b \le 6.0$, more preferably $0.40 \le b \le 5.0$, more preferably $0.50 \le b \le 4.0$, more preferably $0.60 \le b \le 3.0$, more preferably $0.70 \le b \le 2.5$, more preferably $0.80 \le b \le 2.0$, more preferably $0.90 \le b \le 1.5$, and most preferably $1.0 \le b \le 1.4$.

The lower limit of c is 0.10, 0.20, 0.30, 0.40, 0.50, 0.60, 0.70, 0.80, or 0.90 in order of desirability and is most desirably 1.0, and the upper limit of c is 5.0, 4.0, 3.0, 2.5, 2.0, or 1.5 in order of desirability and is most desirably 1.4. That is, the range of c is preferably $0.10 \leq c \leq 5.0$, more preferably $0.20 \leq c \leq 5.0$, more preferably $0.30 \leq c \leq 5.0$, more preferably $0.40 \leq c \leq 5.0$, more preferably $0.50 \leq c \leq 4.0$, more preferably $0.60 \leq c \leq 3.0$, more preferably $0.70 \leq c \leq 2.5$, more preferably $0.80 \leq c \leq 2.0$, more preferably $0.90 \leq c \leq 1.5$, and most preferably $1.0 \leq c \leq 1.4$.

The lower limit of d is 0.11, 0.15, 0.18, 0.20, 0.25, 0.30, or 0.35 in order of desirability and is most desirably 0.40, and the upper limit of d is 9.0, 8.0, 7.0, 6.0, 5.0, 4.0, 3.0, 2.5, 2.0, 1.5, or 1.0 in order of desirability and is most desirably 0.70. That is, the range of d is preferably $0.11 \leq d \leq 9.0$, more preferably $0.11 \leq d \leq 8.0$, more preferably $0.11 \leq d \leq 7.0$, more preferably $0.11 \leq d \leq 6.0$, more preferably $0.11 \leq d \leq 5.0$, more preferably $0.15 \leq d \leq 4.0$, more preferably $0.18 \leq d \leq 3.0$, more preferably $0.20 \leq d \leq 2.5$, more preferably $0.25 \leq d \leq 2.0$, more preferably $0.30 \leq d \leq 1.5$, more preferably $0.35 \leq d \leq 1.0$, and most preferably $0.40 \leq d \leq 0.70$.

The upper limit of e is 0.40, 0.30, 0.20, or 0.10 in order of desirability. That is, the range of e is $0 \leq e \leq 0.40$, $0 \leq e \leq 0.30$, or $0 \leq e \leq 0.20$ in order of preference, and the most preferable range is $0 \leq e \leq 0.10$.

The upper limit of f is 0.80, 0.50, 0.20, or 0.15 in order of desirability and is most desirably 0.10. That is, the range of f is $0 \leq f \leq 0.80$, $0 \leq f \leq 0.50$, $0 \leq f \leq 0.20$, or $0 \leq f \leq 0.15$ in order of preference, and the most preferable range is $0 \leq g1 \leq 0.10$.

The upper limit of g is 5.0, 4.0, 3.0, 2.0, or 1.0 in order of desirability. That is, the range of g is $0 \leq g \leq 5.0$, $0 \leq g \leq 4.0$, $0 \leq g \leq 3.0$, or $0 \leq g \leq 2.0$ in order of preference, and the most preferable range is $0 \leq g \leq 1.0$.

Here, a case in which e, f, and g are 0 is a particularly preferable aspect.

When the catalyst of the present invention is used in a reaction that produces a corresponding unsaturated carboxylic acid from an unsaturated aldehyde such as acrolein and methacrolein as a raw material, particularly in a reaction that produces acrylic acid by subjecting acrolein to catalytic gas-phase oxidation with molecular oxygen or a molecular oxygen-containing gas, improvement in the catalytic activity and reduction in the differential pressure can be achieved, and the use is very effective, as compared with a known method. Also in a process of a partial oxidation reaction accompanied by heat generation, the effect of improving the stability due to reduction of the hot spot temperature or the like can be expected. Further, the catalyst of the present invention is also effective in reducing byproducts that adversely influence the environment and the quality of the final product, such as carbon monoxide (CO), carbon dioxide ($CO_2$), acetaldehyde, acetic acid, and formaldehyde.

[Method for Producing Catalyst, etc.]

Specific steps for obtaining the catalyst according to the present invention are exemplified below.

Step a) preparation

Examples of the raw materials of the elements constituting the catalyst include those shown below.

When ammonium molybdate is used as a molybdenum component raw material, a high-performance catalyst can be obtained.

As raw materials for tungsten, vanadium, antimony, copper and other elements, typically, an oxide, or an ammonium salt, a carbonate salt, an organic acid salt, and a hydroxide, which can be converted into an oxide by high heat, or mixtures thereof can be used. For example, a vanadium component raw material, a molybdenum component raw material, and an antimony component raw material are mixed in a desired ratio with a separately prepared aqueous solution or slurry of a tungsten component raw material and a Z component raw material under conditions of 20 to 95°C, and the mixture is heated and stirred under conditions of 20 to 95°C for about one hour. Then, an aqueous solution in which a copper component raw material is dissolved and, if necessary, an X component raw material and a Y component raw material are added to obtain an aqueous solution or slurry containing the catalyst components. Hereinafter, the aqueous solution or the slurry obtained in this manner is collectively referred to as a prepared liquid (A). Here, the prepared liquid (A) is not always necessary to contain all the catalyst constituent elements, and some elements or some amounts thereof may be added in the subsequent steps. In addition, in the case of adding an amount of water for dissolving the component raw materials or an acid such as sulfuric acid, nitric acid, hydrochloric acid, tartaric acid, and acetic acid for dissolution when preparing the prepared liquid (A), unless the sufficient acid concentration in the aqueous solution to dissolve the raw materials is suitable, for example, in the range of 5 mass% to 99 mass%, the prepared liquid (A) sometimes becomes in the form of a clay-like lump, and this does not result in an excellent catalyst. Therefore, the form of the obtained prepared liquid (A) is preferably an aqueous solution or slurry as an excellent catalyst can be obtained.

Step b) drying

Next, the prepared liquid (A) obtained above is dried to form a dry powder. The drying method is not limited as long as it is a method capable of completely drying the prepared liquid (A), and examples thereof include drum drying, freeze drying, spray drying, and evaporation to dryness. Among these, in the present invention, spray drying is particularly preferred since the slurry can be dried into a powder or granules in a short period of time. The drying temperature in the

EP 4 684 876 A1

spray drying varies depending on a concentration of the slurry, a liquid feeding rate, or the like, and the temperature at the outlet of a dryer is typically 70°C to 150°C. In addition, drying is preferably performed such that the average particle diameter of the dry powder obtained at this time is 20 to 700 μm. In this way, a dry powder (B) is obtained.

Step c) preliminary calcination

When the obtained dry powder (B) is calcined under air circulation at 200°C to 500°C, preferably at 300°C to 400°C, the formability, the mechanical strength, and the catalyst performance of the catalyst tend to improve. The calcination time is preferably 1 hour to 12 hours. In this way, a preliminarily calcined product (C) is obtained.

Step d) pulverization

The obtained preliminarily calcined product (C) is obtained as a solid matter (D) in which the dry powder (B) is aggregated by preliminary calcination. The solid matter (D) is pulverized to obtain a preliminarily calcined powder (E) required in the next forming step. The pulverization method is not limited, but examples thereof include a roller mill, a jet mill, a hammer mill, a rotary mill, and a vibration mill. The average particle diameter (pre-calcined median diameter) of the preliminarily calcined powder (E) obtained at this time is preferably 50 μm or less, more preferably 40 μm or less, further preferably 30 μm or less, and particularly preferably 25 μm or less.

In the present description of this application, the preliminarily calcined powder (E) after pulverization is described as a catalyst precursor, but when there is no aggregation in the stage of the preliminarily calcined product (C) and the preliminarily calcined product (C) can be used without going through the pulverization step, the preliminarily calcined product (C) may be used as a catalyst precursor.

Step e) forming

The forming method is not particularly limited, but in the case of forming into a cylindrical shape or a ring shape, a method using a tablet forming machine, an extrusion forming machine, or the like is preferred. A case of forming into a spherical shape is further preferable, and the preliminarily calcined powder (E) may be formed into a spherical shape using a forming machine, but a method of carrying the preliminarily calcined powder (E) (containing a forming aid and a strength improver if necessary) on a carrier such as inert ceramic is preferable. Here, as a carrying method, a tumbling granulation method, a method using a centrifugal fluid coating device, a wash coating method, and the like are widely known, and the method is not particularly limited as long as the preliminarily calcined powder (E) can be uniformly carried on the carrier. In consideration of the production efficiency of the catalyst and the performance of the prepared catalyst, more preferred is a method of rotating a flat or uneven disc at a high speed in an apparatus that includes the disc at a bottom portion of a fixed cylindrical container, to vigorously stir a carrier charged in the container by rotation and revolution movements of the carrier itself, and carrying the powder component on the carrier by adding, to the container, the preliminarily calcined powder (E), and if necessary a molding aid and/or a strength improver, and a pore-forming agent. A binder is preferably used for carrying. Specific examples of the binder to be used include water, ethanol, methanol, propanol, a polyhydric alcohol, polyvinyl alcohol that is a polymer-based binder, and a silica sol aqueous solution that is an inorganic binder. Ethanol, methanol, propanol, and a polyhydric alcohol are preferred, a diol such as ethylene glycol and a triol such as glycerin are more preferred. When an appropriate amount of an aqueous glycerin solution is used, the formability becomes excellent, and a high-performance catalyst having high mechanical strength can be obtained. Specifically, a particularly high-performance catalyst can be obtained when an aqueous solution of glycerin having a concentration of 5 mass% or more is used. The amount of these binders to be used is typically 2 to 80 parts by mass with respect to 100 parts by mass of the preliminarily calcined powder (E). An inert carrier of about 2 to 8 mm is typically used, and the preliminarily calcined powder (E) is carried thereon. The carrying ratio is determined in consideration of the catalyst usage conditions, for example, reaction conditions such as the space velocity of the reaction raw materials and the concentrations of the raw materials and is typically 20 mass% to 80 mass%. Here, the carrying ratio is expressed as in the following equation (4) when the forming aid, the strength improver, and the like used in forming are included. Thus, a formed body (F) is obtained.

In addition, as found by the present inventors, it is difficult to maintain the mechanical strength in the present invention. Therefore, it is preferable to add inert inorganic fibers as a strength improver during carrying and forming. The amount of the fibers to be used is typically 1 to 30 parts by mass with respect to 100 parts by mass of the preliminarily calcined powder (E).

[Carrying]

The catalyst in which the preliminarily calcined powder, which is obtained by preliminary calcination after preparation of the catalytically active component, or the preliminarily calcined powder after pulverization, which is obtained by further subjecting the preliminarily calcined powder to the pulverization step, is carried on the inert carrier has a particularly excellent effect.

As the material of the inert carrier, known materials such as alumina, silica, titania, zirconia, niobia, silica-alumina, silicon carbide, carbide, and a mixture thereof can be used. The particle diameter, the water absorption, the mechanical strength, the crystallinity and the mixing ratio of each crystal phase, and the like of the inert carrier are not particularly limited, and appropriate ranges should be selected in consideration of the final catalyst performance, the formability, the production efficiency, and the like. The shape of the inert carrier is preferably spherical but is not particularly limited, and a pellet, a ring, or the like is used. The mixing proportion of the carrier and the preliminarily calcined powder is typically calculated as a carrying ratio based on the charged mass of each raw material according to the following equation (4). When it is clear that the additives such as the forming aid and the strength improver used remain in the catalyst even after the main calcination, the additives are included in the total amount (denominator).

[Equation (4)]

$$\text{Carrying ratio (mass\%)} = \text{(mass of preliminarily calcined powder used for forming)}/\{\text{(mass of preliminarily calcined powder used for forming)} + \text{(mass of carrier used for forming)}\} \times 100 \tag{4}$$

The upper limit of the carrying ratio is preferably 80 mass% and is 70, 60, 55, 50, 45, or 40 mass% in order of preference.

The lower limit thereof is preferably 10 mass% and is 15, 18, or 20 mass% in order of preference. That is, the carrying ratio is preferably 10 mass% or more and 80 mass% or less, more preferably 10 mass% or more and 70 mass% or less, more preferably 10 mass% or more and 60 mass% or less, more preferably 10 mass% or more and 55 mass% or less, more preferably 15 mass% or more and 50 mass% or less, and more preferably 18 mass% or more and 45 mass% or less, and the most preferable range is 20 mass% or more and 40 mass% or less.

[Inorganic Fiber]

The catalyst of the present invention preferably contains inorganic fibers for the purpose of improving the mechanical strength or the like. The material of the inorganic fibers is not particularly limited, but for example, glass fibers, ceramic fibers, metal fibers, mineral fibers, carbon fibers, various whiskers, and the like can be used. Of these, glass fibers treated with a silane-based chemical product are particularly preferable.

The fiber length thereof is not particularly limited as long as it does not impair the effects of the present invention, but the average fiber length is preferably about 1 to 1000 $\mu$m, and more preferably about 10 to 500 $\mu$m.

Further, two or more types of the inorganic fibers can be used in combination, and two or more types having different materials or two types of a same material having different average fiber lengths may be used.

Step f) main calcination

When the formed body (F) is calcined at a temperature of 100 to 450°C for about 1 to 12 hours, the catalytic activity and the effective yield tend to improve. The calcination temperature is preferably 270°C or higher and 420°C or lower, and more preferably 350°C or higher and 400°C or lower. Air is convenient and preferred as the gas to be circulated. In addition, nitrogen, carbon dioxide, argon, helium, a nitrogen oxide-containing gas for creating a reducing atmosphere, an ammonia-containing gas, a hydrogen gas, and a mixture thereof can be also used as the inert gas. In this way, a catalyst (G) is obtained.

[Method for Measuring Surface Roughness S in Spherical Catalyst]

In the measurement of the surface roughness S of the catalyst formed body, in order to measure an accurate value, it is necessary to measure a certain distance while the value of the surface roughness S measured is prevented from becoming larger than the actual surface roughness due to the shape of the formed body. That is, when a formed body having a completely smooth surface is measured, it is necessary to determine the measurement position in such a manner that the surface height Rz which is a value obtained by subtracting the height of the lowest point from the height of the highest point of all the measurement points becomes 0. For example, when the formed body has a cylindrical shape or a columnar shape, the side surface of the formed body is measured along the axial direction. The measurement distance L in this case is set to, for example, 1/3 to 2/3 of the length in the axial direction of the formed body.

When the shape of the formed body is spherical, it is particularly difficult to distinguish between the unevenness of the surface and the roundness of the sphere when the laser for measuring the height is moved in the horizontal direction, and arbitrary properties are likely to occur even when the correction is performed. In order to avoid this, the spherical formed body is widely observed to find out the highest position of the formed body, and a circle having a circumferential length that is 1/3 to 2/3 of the catalyst particle diameter is drawn around the highest position of the formed body as the center in a plan view. Points on the surface of the formed body that overlap with the circle in the plan view ideally have the same height when the formed body has a completely smooth surface. Therefore, when the height is measured along the circumference of the circle, it is possible to avoid the influence of the roundness of the sphere on the height. When the measurement is made along the circumference of the circle with a measurement distance L set to be equal to the length of the circumference of the circle, the surface height Rz needs to be 150 $\mu$m or less and is preferably 100 $\mu$m or less.

When the formed body is a cylindrical or columnar extrusion catalyst, the side surface of the formed body may be measured along the extrusion axis direction.

In this regard, for example, when a shape analysis laser microscope VK-X1000 (manufactured by KEYENCE) is used, after determining a measurement range including the spherical formed body using an objective lens that magnifies five times and focusing, the measurement and the analysis above are automatically performed by the laser microscope, and the value of the surface roughness S of the formed body can be obtained. The laser used in the measurement is, for example, a red semiconductor laser having a wavelength of 661 nm and a maximum output of 1 mW.

[Method for Adjusting Surface Roughness S]

The value of the surface roughness S can be adjusted by changing the raw materials used in the step a), the spray drying condition in the step b), the calcination temperature and time in the step c), the pulverization method and the median diameter of the preliminarily calcined powder (E) after pulverization in the step d), and the relative centrifugal acceleration, the carrying ratio, the type of the binder, the addition position of the binder, and the like in the step e). However, it is difficult to greatly change the value by changing a single condition, and the value can be suitably adjusted by optimizing two or more conditions. Some examples will be described below.

<Calcination Temperature and Time in Step c)>

The calcination temperature is preferably 200°C to 500°C as described above, but when the calcination temperature is increased, the surface roughness S tends to increase. Therefore, for adjusting the surface roughness S, the calcination temperature is preferably lower than 400°C. Further, because the surface roughness S may also increase as the calcination time becomes longer, the calcination time is preferably shorter than five hours.

<Pulverization Method, Median Diameter, and Centrifugal Acceleration in Step d)>

The surface roughness S can also be controlled by the method of the step d). When a ball mill is used, the surface roughness S is easily adjusted, which is preferable. The surface roughness S can also be adjusted by adjusting the median diameter of the preliminarily calcined powder (E), and the median diameter of the preliminarily calcined powder (E) is preferably 50 $\mu$m or less. When the step e) is granulation, the surface roughness S can also be adjusted by changing the relative centrifugal acceleration during granulation according to the median diameter of the preliminarily calcined powder (E). Specifically, when the median diameter of the preliminarily calcined powder (E) is larger than 30 $\mu$m, the surface roughness S tends to fall within a desirable range by adjusting the relative centrifugal acceleration during granulation to 10 G or less.

<Relative Centrifugal Acceleration and Temperature and Humidity Ratio in Granulator in Step e)>

When the step e) is granulation, the value of the surface roughness S can also be adjusted by changing the relative centrifugal acceleration and the temperature and the absolute humidity in the granulator. The relative centrifugal acceleration may be about 2.0 G or more and 30 G or less, but when the relative centrifugal acceleration is increased, the surface roughness S tends to be low. However, it depends also on the humidity ratio in the granulator. The temperature in the granulator is preferably 5°C or higher and 50°C or lower. The humidity ratio in the granulator is preferably 0.0235 kg-H$_2$O/kg-DA or less. The upper limit of the humidity ratio is preferably 0.0230 kg-H$_2$O/kg-DA and is 0.0220, 0.0200, 0.0190, 0.0180, or 0.0175 kg-H$_2$O/kg-DA in order of preference. The lower limit of the humidity ratio is preferably 0 kg-H$_2$O/kg-DA and is 0.0010, 0.0020, 0.0030, 0.0040, 0.0050, 0.0060, 0.0070, 0.0100, 0.0125, 0.0150, or 0.0160 kg-H$_2$O/kg-DA in order of preference. That is, the humidity ratio is preferably 0 kg-H$_2$O/kg-DA or more and 0.0235 kg-H$_2$O/kg-DA or less, more preferably 0.0010 kg-H$_2$O/kg-DA or more and 0.0235 kg-H$_2$O/kg-DA or less, more preferably 0.0020 kg-H$_2$O/kg-DA or more and 0.0235 kg-H$_2$O/kg-DA or less, more preferably 0.0030 kg-H$_2$O/kg-DA or more and 0.0235 kg-H$_2$O/kg-DA or less, more preferably 0.0040 kg-H$_2$O/kg-DA or more and 0.0235 kg-H$_2$O/kg-DA or less, more preferably 0.0050 kg-H$_2$O/kg-DA or more and 0.0235 kg-H$_2$O/kg-DA or less, more preferably 0.0060 kg-H$_2$O/kg-DA or more and 0.0230 kg-H$_2$O/kg-DA or less, more preferably 0.0070 kg-H$_2$O/kg-DA or more and 0.0220 kg-H$_2$O/kg-DA or less, more preferably 0.0100 kg-

$H_2O$/kg-DA or more and 0.0200 kg-$H_2O$/kg-DA or less, more preferably 0.0125 kg-$H_2O$/kg-DA or more and 0.0190 kg-$H_2O$/kg-DA or less, more preferably 0.0150 kg-$H_2O$/kg-DA or more and 0.0180 kg-$H_2O$/kg-DA or less, and particularly preferably 0.0160 kg-$H_2O$/kg-DA or more and 0.0175 kg-$H_2O$/kg-DA or less.

The temperature and the humidity ratio in the granulator above are different from the temperature and the humidity in the room where granulation operation is performed and mean the temperature and the humidity around the bottom board of the granulator. The humidity ratio may be obtained by measuring a point with a height of 10 cm from the bottom board on the rotation axis of the bottom board of the granulator after waiting until the temperature and the humidity are stabilized after the start of the granulation operation (for example, after 15 minutes or longer). Examples of the methods for controlling the temperature and the absolute humidity in the granulator include, but are not limited to, control of the evaporation amount of water derived from the binder, control of the granulator temperature with a jacket, control of the volume of the supplied air or the exhaust air, control of the room temperature and the humidity in the granulation chamber, atmosphere control in which a lid or a cover is installed in the upper part of the granulator, and the like.

[Method for Calculating Humidity Ratio]

The humidity ratio, which is the ratio of the mass of water vapor to the mass of dry air, may be measured with a commercial absolute hygrometer or may be determined by converting the temperature T (°C) and the relative humidity RH (%) measured with a relative hygrometer to the humidity ratio MR (kg-$H_2O$/kg-DA) by the following equation (B).
[Equation (B)]

$$MR=0.622*(6.1078*10^{\wedge}(7.5*T/(T+237.3))*RH/100)/(1013.25- (6.1078*10^{\wedge}(7.5*T/(T+237.3))*RH/100)) \qquad (B)$$

[Use of Catalyst]

In the method for producing acrylic acid using the catalyst of the present invention, the method for circulating the raw material gas may be a normal single flow method or a recycling method, and the method can be carried out under generally used conditions and is not particularly limited. For example, a mixed gas containing 1 to 10 vol% and preferably 4 to 9 vol% of a starting raw material substance as an ideal gas, 3 to 20 vol% and preferably 4 to 18 vol% of molecular oxygen, 0 to 60 vol% and preferably 4 to 50 vol% of water vapor, and 20 to 80 vol% and preferably 30 to 60 vol% of an inert gas such as carbon dioxide and nitrogen is introduced, at 200 to 450°C under normal pressure to pressure of 10 atm at a space velocity of 300 to 5000 h$^{-1}$, into the catalyst of the present invention filled in a reaction tube, and the reaction is carried out.

In the method for producing acrylic acid, one type of catalyst may be used alone, or different types of catalysts may be used for multilayer filling depending on the conditions to be used. That is, in an adoptable method, a plurality of catalyst layers divided and formed in the direction of the raw material gas flow of the reaction tube are provided, and the plurality of types of catalysts are arranged in such a manner that the activity increases from the raw material inlet portion toward the outlet portion in the direction of the raw material gas flow. In the case of using a reaction tube filled with two or more types of catalysts in multiple layers, at least one type thereof may be the catalyst of the present invention, but it is preferable that two or more types of catalysts satisfying the requirements of the present invention are filled. The number of the divided catalyst layers is not particularly limited but is typically two to five, and preferably two or three. The different types of catalysts not only mean a case where the compositions of the catalysts are different alone but also include a case where the carrying ratios to the inert carrier are different or a case where the dilution rates are different. In addition, a method in that the catalyst of the present invention, which is highly activated by adjusting the surface roughness S, is disposed on the raw material gas outlet side and in that a catalyst that is relatively low in activity is disposed on the raw material gas inlet side can also be adopted.

EXAMPLES

Hereinafter, the present invention will be described in more detail with reference to Examples. In the Examples, the raw material conversion rates and the selectivity were calculated according to the following equations.

Raw material conversion rate (%) = (number of moles of reacted acrolein/number of moles of supplied acrolein) $\times$ 100

Selectivity (%) = (number of moles of produced acrylic acid)/(number of moles of reacted acrolein) $\times$ 100

Although Examples are shown below by giving specific examples, the present invention is not limited to Examples unless it deviates from the spirit thereof. The median diameters of the preliminarily calcined powders (E) shown in the Examples are median values in a volume fraction measured using a particle size distribution measuring device LMS-2000e manufactured by Seishin Enterprise Co., Ltd. The measurement conditions were conditions of measuring with a scattering range of 10 to 20%, measurement time of three seconds, and the number of snaps of 3000, using Fraunhofer as the sample material and water as the dispersion medium, by setting the stirrer pump at 2500 rpm without applying ultrasonic waves.

The temperature and the humidity (relative humidity) in the granulator shown in the Examples were measured at a point with a height of 10 cm from the bottom board on the rotation axis of the bottom board of the granulator 15 minutes after the start of the granulation operation using a thermo-hygrometer PC-5120 manufactured by Sato Keiryoki Mfg. Co., Ltd. From the temperature and the relative humidity obtained by the measurement, the humidity ratio was calculated using the equation (B).

The surface roughness S of each catalyst shown in the Examples was measured and analyzed using a shape analysis laser microscope VK-X1000 (manufactured by KEYENCE). Specifically, the measurement was performed using a red semiconductor laser having a wavelength of 661 nm and a maximum output of 1 mW by focusing on the catalyst to be measured using an objective lens that magnified five times. In setting the measurement distance L, the average value of the values obtained by measuring the particle diameters of 100 particles of the catalyst with a caliper was taken as the catalyst particle diameter, and the measurement distance L was set in the range of 1/3 to 2/3 of the catalyst particle diameter.

The analysis was made under conditions not setting the cut-off, and the arithmetic average of the measurement of 10 formed body was defined as the surface roughness S.

[Example 1]

<Production of Catalyst 1>

Ammonium molybdate, ammonium paratungstate, ammonium metavanadate, copper sulfate, and antimony acetate were weighed in such a manner that the composition of the catalytically active component became $Mo_{12}V_{3.0}W_{1.2}Cu_{1.2}Sb_{0.50}$ and mixed in a water solvent heated to 95°C to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 21.6 $\mu$m. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for forming, and the centrifugal acceleration was 26.0 G. The humidity ratio in the granulator was 0.0169 kg-$H_2$O/kg-DA, and the temperature was 27.1°C.

Next, main calcination was performed under the conditions of 390°C and four hours to obtain a spherical catalyst 1 of the present invention.

[Example 2]

<Production of Catalyst 2>

Ammonium molybdate, ammonium paratungstate, ammonium metavanadate, copper sulfate, and antimony acetate were weighed in such a manner that the composition of the catalytically active component became $Mo_{12}V_{3.0}W_{1.2}Cu_{1.2}Sb_{0.50}$ and mixed in a water solvent heated to 95°C to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 37.9 $\mu$m. To the preliminarily calcined powder (E), 5 mass% of crystalline cellulose was added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for forming, and the centrifugal acceleration was 4.2 G. The humidity ratio in the granulator was 0.0069 kg-$H_2$O/kg-DA, and the temperature was 21.2°C.

Next, main calcination was performed under the conditions of 390°C and four hours to obtain a spherical catalyst 2 of the present invention.

[Example 3]

<Production of Catalyst 3>

Ammonium molybdate, ammonium paratungstate, ammonium metavanadate, copper sulfate, and antimony acetate were weighed in such a manner that the composition of the catalytically active component became $Mo_{12}V_{3.0}W_{1.2}Cu_{1.2}Sb_{0.50}$ and mixed in a water solvent heated to 95°C to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 36.7 $\mu$m. To the preliminarily calcined powder (E), 5 mass% of crystalline cellulose was added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for forming, and the centrifugal acceleration was 4.2 G. The humidity ratio in the granulator was 0.0170 kg-$H_2$O/kg-DA, and the temperature was 31.0°C.
Next, main calcination was performed under the conditions of 390°C and four hours to obtain a spherical catalyst 3 of the present invention.

[Comparative Example 1]

<Production of Catalyst 4>

Ammonium molybdate, ammonium paratungstate, ammonium metavanadate, copper sulfate, and antimony acetate were weighed in such a manner that the composition of the catalytically active component became $Mo_{12}V_{3.0}W_{1.2}Cu_{1.2}Sb_{0.50}$ and mixed in a water solvent heated to 95°C to obtain a prepared liquid (A). The prepared liquid (A) was dried by a spray drying method, and the obtained dry powder (B) was preliminarily calcined under the conditions of 350°C and four hours to obtain a preliminarily calcined product (C). The obtained preliminarily calcined product (C) was pulverized with a vibration mill to obtain a preliminarily calcined powder (E). The median diameter of the obtained preliminarily calcined powder (E) was 21.8 $\mu$m. To the preliminarily calcined powder (E), 5 mass% of crystalline cellulose and 5 mass% of Milled Fiber EFH150-31 manufactured by Central Glass Co., Ltd. were added and mixed thoroughly, and then the obtained product was carried and formed into a spherical shape on an inert spherical carrier made of a mixture of silica and alumina in such a manner that the carrying ratio became 33 mass% and that the average value of the particle diameters became 5 mm by a tumbling granulation method using a 20 mass% glycerin solution as a binder. A tumbling granulator was used for forming, and the centrifugal acceleration was 26.0 G. The humidity ratio in the granulator was 0.0235 kg-$H_2$O/kg-DA, and the temperature was 27.9°C.
Next, main calcination was performed under the conditions of 390°C and four hours to obtain a spherical catalyst 4 of the present invention.
Oxidation reaction was carried out using the obtained catalysts 1 to 4. A reaction tube having an inner diameter of 28.4 mm was filled with 67.6 ml of the catalyst, and a gas having the following composition in which oxygen and nitrogen were added to a gas obtained by catalytic gas-phase oxidation of propylene using a molybdenum-bismuth-based catalyst was introduced. A reaction was performed at SV (space velocity; flow rate of raw material gas per unit time/apparent volume of filled catalyst) of 1020/hr and reaction bath temperature of 260°C.

| | |
|---|---|
| Acrolein | 5.9 vol% |
| Unreacted propylene + other organic compound | 1.7 vol% |
| Oxygen | 4.7 vol% |
| Steam | 17.2 vol% |
| Nitrogen-containing inert gas | 70.5 vol% |

The reaction results obtained by the oxidation reaction and the results obtained by measuring the surface roughness are shown in Table 1.

[Table 1]

| Catalyst Name | Raw Material Conversion Rate (%) | Selectivity (%) | Surface Roughness S ($\mu$m) | Surface Height Rz ($\mu$m) | Measurement Distance L ($\mu$m) |
|---|---|---|---|---|---|
| Catalyst 1 | 99.1 | 96.9 | 12.6 | 69.4 | 2448.1 |
| Catalyst 2 | 98.7 | 97.5 | 19.6 | 97.9 | 2887.1 |
| Catalyst 3 | 98.5 | 97.6 | 22.1 | 94.6 | 2076.4 |
| Catalyst 4 | 96.6 | 97.1 | 11.6 | 68.8 | 2491.2 |

From the results in Table 1, it was found that the catalysts of the present invention had a high raw material conversion rate, namely, a high catalytic activity.

The present application is based on Japanese Patent Application No. 2023-046098 filed on March 23, 2023, contents of which are incorporated herein by reference.

INDUSTRIAL APPLICABILITY

According to the present invention, a catalyst which has an excellent catalytic activity when an unsaturated carboxylic acid is produced by subjecting an unsaturated aldehyde as a raw material to a catalytic gas-phase oxidation reaction can be provided. Therefore, this allows a plant for producing acrylic acid to be stably operated for a long period of time, which is very useful.

**Claims**

1. A catalyst for producing an unsaturated carboxylic acid comprising a catalytically active component with a composition represented by the following formula (1),

   wherein a value of surface roughness S obtained by measuring a surface roughness of the catalyst with a non-contact shape-measuring laser microscope is 11.7 $\mu$m or more and 25.0 $\mu$m or less,

   $$(Mo)_{12}(V)_a(W)_b(Cu)_c(Sb)_d(X)_e(Y)_f(Z)_g(O)_h \qquad (1),$$

   where, in the formula (1), Mo, V, W, Cu, Sb, and O represent molybdenum, vanadium, tungsten, copper, antimony, and oxygen respectively; X represents at least one element selected from the group consisting of an alkali metal and thallium; Y represents at least one element selected from the group consisting of magnesium, calcium, strontium, barium, and zinc; Z represents at least one element selected from the group consisting of niobium, cerium, tin, chromium, manganese, iron, cobalt, samarium, germanium, titanium, and arsenic; a, b, c, d, e, f, g, and h represent the atomic ratios of the respective elements; a satisfies $0 < a \leq 10.0$, b satisfies $0 \leq b \leq 10.0$, c satisfies $0 < c \leq 6.0$, d satisfies $0 \leq d \leq 10.0$, e satisfies $0 \leq e \leq 0.50$, f satisfies $0 \leq f \leq 1.0$, and g satisfies $0 \leq g < 6.0$, with respect to molybdenum atom being 12; and h is the number of oxygen atoms necessary for satisfying the valence of each component.

2. The catalyst for producing an unsaturated carboxylic acid according to claim 1, wherein in the formula (1), $1.0 \leq a \leq 5.0$, $0.50 \leq b \leq 3.0$, $0.50 \leq c \leq 3.0$, and $0 < d \leq 2.0$ are satisfied.

3. The catalyst for producing an unsaturated carboxylic acid according to claim 1 or 2, wherein the catalytically active component is carried on an inert carrier.

4. The catalyst for producing an unsaturated carboxylic acid according to claim 3, wherein the inert carrier is silica, alumina, or a combination thereof.

5. A method for producing the catalyst for producing an unsaturated carboxylic acid according to claim 1 or 2, comprising performing granulation by a granulator, wherein the humidity ratio MR in the granulator is 0.0235 kg-$H_2$O/kg-DA or less.

6. A method for producing an unsaturated carboxylic acid using the catalyst for producing an unsaturated carboxylic acid according to claim 1 or 2.

7. A method for producing an unsaturated carboxylic acid using a reaction tube filled with two or more types of the catalyst for producing an unsaturated carboxylic acid according to claim 1 or 2 in multiple layers.

## INTERNATIONAL SEARCH REPORT

| International application No. |
| --- |
| **PCT/JP2024/008478** |

**A. CLASSIFICATION OF SUBJECT MATTER**

*B01J 35/60*(2024.01)i; *B01J 23/888*(2006.01)i; *B01J 37/00*(2006.01)i; *C07C 51/235*(2006.01)i
FI:    B01J35/60; B01J23/888 Z; B01J37/00 E; C07C51/235

According to International Patent Classification (IPC) or to both national classification and IPC

**B. FIELDS SEARCHED**

Minimum documentation searched (classification system followed by classification symbols)

B01J35/60; B01J23/888; B01J37/00; C07C51/235

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2018/051933 A1 (NIPPON KAYAKU KABUSHIKI KAISHA) 22 March 2018 (2018-03-22)<br>claims 1-2, paragraph [0009] | 1-7 |
| A | WO 2021/193774 A1 (NIPPON KAYAKU KABUSHIKI KAISHA) 30 September 2021 (2021-09-30)<br>claims 1-14, paragraph [0042] | 1-7 |
| A | JP 2007-117818 A (ASAHI KASEI CHEMICALS CORPORATION) 17 May 2007 (2007-05-17)<br>claims 1-9, paragraph [0027] | 1-7 |
| A | JP 2016-536136 A (BASF SE) 24 November 2016 (2016-11-24)<br>entire text | 1-7 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **25 April 2024** | **14 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

<div align="center">

**INTERNATIONAL SEARCH REPORT**
Information on patent family members

</div>

| International application No. |
| --- |
| **PCT/JP2024/008478** |

| Patent document cited in search report | | | Publication date (day/month/year) | Patent family member(s) | | | Publication date (day/month/year) |
| --- | --- | --- | --- | --- | --- | --- | --- |
| WO | 2018/051933 | A1 | 22 March 2018 | US 2019/0262806 A1 claims 1-2, paragraphs [0016], [0017] | | | |
| | | | | EP 3513874 A1 | | | |
| | | | | CN 109715290 A | | | |
| | | | | KR 10-2019-0046853 A | | | |
| WO | 2021/193774 | A1 | 30 September 2021 | US 2023/0143920 A1 claims 1-14, paragraph [0149] | | | |
| | | | | EP 4112554 A1 | | | |
| | | | | CN 115515902 A | | | |
| JP | 2007-117818 | A | 17 May 2007 | (Family: none) | | | |
| JP | 2016-536136 | A | 24 November 2016 | US 2015/0080605 A1 entire text | | | |
| | | | | WO 2015/039982 A1 | | | |
| | | | | EP 3046668 A1 | | | |
| | | | | KR 10-2016-0056893 A | | | |
| | | | | CN 106687212 A | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H08299797 A
- JP 2003251184 A
- JP 2015120133 A
- JP 2001079408 A
- WO 2012073584 A
- JP 2009214105 A
- JP 2015096497 A
- WO 2020196150 A
- JP 2023046098 A